# EUROPEAN PATENT APPLICATION

(11) **EP 4 653 092 A1**
(43) Date of publication of application: **26.11.2025**
(21) Application number: 25171756.7
(22) Date of filing: 22.04.2025
(51) Int. Cl.: B01L 3/00

(54) **TEST TUBE INSERTABLE SPERMATOZOA SEPARATION DEVICE**

(30) Priority: 18.04.2024 US 202463635920 P; 15.04.2025 US 202563788944 P
(71) Applicant: Reprohealth Technologies, Inc., Indianapolis, IN 46256 (US)
(72) Inventor: DONAHUE, James G, Indianapolis, 46228 (US); TKATCHOUK, Alexander A., Huntington Beach, 92647 (US); WHEELER, Reese, Grass Valley, 95945 (US); TODIO, Charles A., Santa Clarita, 91351 (US); HOPKINS, Zoe L., Aurora, 80016 (US); BAHR, Otto, Visalia, 93277 (US); TALIAFERRO, Lucas, Denver, 80246 (US); WALKER, William R., Bethesda, 20817 (US)
(74) Representative: V.O.

(57) **Abstract**

A motility-based spermatozoa separation device has a hollow cylindrical body that includes an internal passageway. The hollow cylindrical body is configured for insertion into a test tube having a semen sample placed therein. A sperm separating member is disposed in the internal passageway. When the hollow cylindrical body is inserted into the test tube, the sperm separating member divides the test tube into an upper cavity and a lower cavity. A suitable media can be inserted in the internal passageway (e.g., the media flowing downwardly through the sperm separating member and onto the semen sample located in the lower cavity) placing the lower cavity in fluid communication with the upper cavity. Progressively motile spermatozoa from the semen sample can swim from the lower cavity toward the sperm separating member, swimming upwardly therethrough, and into the upper cavity to be obtained therefrom.

## Description

### Cross-Reference to Related Applications

The present application claims the benefit of U.S. Provisional Application No. 63/635,920, filed April 18, 2024, and U.S. Provisional Application No. 63/788,944, filed April 15, 2025, the entirety of which are herein incorporated by reference.

### Technical Field

The present application generally relates to separation of spermatozoa, and more specifically, but not exclusively, to test tube insertable devices for swim-up motility based separation of spermatozoa.

### Background

It is estimated that twenty percent (20%) to thirty percent (30%) of infertility cases are due to male infertility (e.g., male producing sperm of insufficient quantity and/or quality). Male infertility can involve low sperm concentration, poor sperm motility, and diminished viability. Asthenozoospermia, a sperm quality defect which is defined by less than for percent (40%) total motile and progressively motile sperm, remains a prevalent concern. Genetics, uro-genital infections, and a variety of behavioral/lifestyle factors (e.g., smoking, high alcohol consumption, etc.) can result in poor sperm quality and male infertility.

Progressively motile sperm are viewed as viable (e.g., likely capable of fertilizing a female gamete, which is often referred to as an ovum or egg). Progressively motile sperm are sperm which move actively, either linearly or in a large circle. Non-progressively motile sperm (e.g., sperm which exhibit any other pattern of movement with an absence of progression) and non-motile sperm (e.g., immotile) have a significantly lower likelihood of fertilizing an ovum. Therefore, progressively motile sperm are desired for use with assisted reproductive technologies (ART).

The selection criteria for procedures like intrauterine insemination (IUI) and In Vitro Fertilization (IVF) hinge sperm motility. For instance, a good candidate for IUI requires at least five million motile sperm, with one million being progressively motile. IVF, constituting 2.1% of live births in 2019, is a prevalent solution for infertility. Artificial insemination approaches, such as IUI and Intracervical Insemination (ICI), cater to diverse fertility challenges. However, specially prepared motile sperm are needed for favorable outcomes (e.g., fertilization, hopefully resulting in pregnancy to term).

Therefore, a key focus for ART is the isolation of motile sperm from semen samples, which is critical for high pregnancy rates in fertility treatments. However, traditional methods of sperm isolation often present challenges, including damage to sperm cells, negatively impacting fertilization and pregnancy success rates. Techniques currently utilized to separate and isolate motile sperm from immotile sperm include swim-up centrifugation and density gradient centrifugation.

Centrifugation swim-up yields highly motile sperm; however, generation of higher intracellular reactive oxygen species can occur during centrifugation. These intracellular reactive oxygen species can increase sperm DNA fragmentation and apoptosis (cell death). On the other hand, density gradient centrifugation offers purified motile sperm with lower DNA fragmentation (e.g., some may be present due to centrifugation) but is significantly more costly and time consuming than centrifugation swim-up.

The Zymot^{®} microfluidic semen separation device attempted to overcome some of these obstacles. However, there are numerous drawbacks with this device which include a high device cost and the overall time a user must spend actively using the device (e.g., not merely the time the device is positioned in the incubator). This device requires that the semen sample be moved from a test tube and inserted into the inlet port of the device with a first syringe. A second syringe must be utilized to extract the motile sperm from the outlet port of the device after around a 30 minute period of incubation. The user must be careful not to draw up the sperm containing fluid from the outlet port too quickly or non-motile sperm can be pulled through the microfluidic channels, reducing the percentage of motile sperm in the sperm containing fluid.

The motile sperm is then transferred from the second syringe into a test tube (e.g., commonly a 15 mL conical tube as is utilized for IVF). As will be appreciated to a person of skill, the multiple transfers required to utilize the Zymot^{®} microfluidic semen separation device are time consuming and can increase the potential for sample contamination.

These current techniques can separate motile sperm in a workmanlike manner, however, they suffer from numerous drawbacks including damage to sperm cells, high cost, require highly specialized training, can require expensive tools and chemical gradients, and are time consuming.

Therefore, further technological developments are desirable.

### Summary

Devices, apparatus, and methods for swim-up separation or sorting of sperm within a test tube are provided, including a test tube insertable semen separating device for separating motile sperm from a semen sample. The separating device allows progressively motile sperm cells to swim-up through pores in a sperm separating filter member and into an upper cavity for collection and use. The separating filter member restricts unhealthy sperm (e.g., immotile and non-progressively motile spermatozoa) from passing into the upper cavity. Advantageously, in addition to restricting non-motile and non-progressively motile sperm, cellular and/or other debris from the semen sample are maintained in the lower cavity, below the separating filter.

Entrance of progressively motile sperm cells into the upper cavity of the test tube provides for ease of extraction (e.g., the progressively motile sperm cells can be drawn up in fluid media with a pipette). The separating device can be configured to fit into a 15 mL conical test tube (e.g., as is commonly utilized for human IVF) or into a 50 mL conical test tube (e.g., as has been discovered to provide a sufficient holding capacity for a typical porcine specimen); however, other configurations are contemplated depending upon the desired semen sample size from which motile sperm are to be sorted. This technology provides a non-invasive, efficient means to isolate motile sperm without compromising sperm integrity, as can occur with centrifugation.

To separate healthy from unhealthy sperm, the filtering device utilizes a filtration method of a filter, microfluidic channels, or mucus-like gel as a physical barrier. The filtering device can also use gradients (chemotaxis, nutrient gradients, and temperature gradients) to promote the movement of healthy sperm cells. These two categories can also be combined (for example, having a nutrient gradient across a filter).

To capture healthy sperm, the filtering device uses a unidirectional barrier, which would create a collection area above the device that prevents sperm from swimming back down into the filter, a pipette to remove the motile sperm from the tube to place into another tube; and microfluidic channels to control the travel of the healthy sperm once they are through the device. These methods are not necessarily mutually exclusive, although use of more than one may be more complex than necessary.

To place the filtering device into the test tube, three methods can be used: a positioning rod, which would allow the clinician to push the device just above the fluid level of the sample; a mechanized push device, which could more precisely control where the device sits within the tube; and an external device that uses magnets both within the device and within the positioner to place the device in the same location in every test tube.

To hold the filtering device in place within the test tube, three methods can be used: an adhesive, which could permanently adhere the device to the tube; and a rubber seal surrounding the device that would sit between the device and the test tube wall; and a silicone suction device.

The present filtering device, apparatus and methods are intended for use by clinicians who are performing semen separation as part of the process of assisted reproductive care with semen from the donor.

The present filtering device causes measurably less damage to the sperm cells than centrifugation and must be sterile before placement in the sample. The present filtering devices have broad applicability for both human and animal sperm motility-based swim-up separation (e.g., bovine, equine, porcine, etc.).

The present filtering device can withstand gamma radiation for device sterilization through radiation, wherein 5-40 kGy is a standard measure for sterilization. The filtering device will be formed of materials known to withstand this sterilization method.

Compatibility with biohazard processing is important to ensure that the entire test tube of unhealthy sperm with the device can be placed in the biohazard waste bin at the clinic rather than being taken apart for different processing. Materials known to withstand biohazard waste processing will be used.

The sperm separating device can be injection molded from a polymer (e.g., crystal polystyrene, polypropylene, etc.) and can be a single-use disposable device. However, the device can be produced through a variety of methods, including additive manufacturing (e.g., 3-D printing), vacuum molding, or other forming processes which are suitable for mass device production of medical devices.

The sperm separating member can take the form of a filter. The filter can be a woven filter or a non-woven membrane, such as an etched membrane. A nylon or polyethylene terephthalate (PET) filter with a pore size of 10 micrometers may be used. Pore sizes ranging from 5 micrometers to 100 micrometers are contemplated to accommodate different species (e.g., different species may require different pore sizes for adequate separation of motile sperm). Etched polycarbonate with a pore size of 10 micrometers may be used.

Requirements fulfilled by the present invention fall into several categories: requirements that ensure health and safety of sperm within sample, requirements that ensure accuracy of device, and requirements that govern ease of use and ease to obtain. The health and safety of the sperm in the separation step is determined by the sterility of the device, preventing use of existing methods that cause damage (such as centrifugation), and ensuring that the resulting device cannot cause damage to the cells.

Accuracy of separation ensures that the device performs the desired function as well or better than existing separation techniques. Ease of use includes the ergonomics of use, time of use, operating steps, and disposal steps, and obtainability of the device is related to affordability and ease of manufacturing. Semen is generally collected and placed in 15 ml polystyrene conical test tubes for further processioning. The sperm separating devices described herein utilize these common disposable test tubes, reduce skilled labor time, typically that of a fertility lab technician (e.g., compared to density gradient centrifugation, swim-up centrifugation, and prior art microfluidic channel devices requiring multiple semen/sperm syringe-based transfers). Additionally, the reduction of semen/sperm transfers can reduce the risk of contamination, mis-labeling, and/or laboratory error.

A test tube insertable spermatozoa separation device includes a housing having a lower portion and an upper portion. The lower portion is configured for insertion into a test tube. A sperm separating member positioned within an internal area of the lower portion of the housing, wherein the sperm separating member is configured to permit motile spermatozoa to swim upwardly therethrough toward the upper portion of the housing.

The sperm separating member restricts the passage of non-motile spermatozoa. An outer portion of the housing can be structured to closesly engage with an inner wall of the test tube.

The housing can include an outer wall defining a cylindrical shell-like form. The internal area can be an internal passageway that extends from the lower portion to the upper portion, and the sperm separating member can be located within the internal passageway.

The outer wall can include a plurality of viewing windows. The sperm separating member can include a plurality of microfluidic channels. The sperm separating member can be a sperm separating filter having a pore size greater than 5 µm and less than 30 µm. The filter is formed of at least one of nylon, polyethylene terephthalate, and polycarbonate.

A test tube insertable spermatozoa swim-up separation device is described which includes a housing having an internal passageway extending therethrough. A lower portion of the housing is configured for insertion into a test tube. A sperm separating filter is located within the internal passageway, and the sperm separating filter is configured to permit motile spermatozoa to swim upwardly therethrough while restricting the passage of non-motile spermatozoa.

The housing can include a hollow cylindrical form. A retention ring can be positioned within the internal passageway, and the retention ring can retain the sperm separating filter in the internal passageway. An outer circumference of the retention ring can engage with the housing.

The sperm separating filter can be located toward the lower portion of the housing. Upon insertion of the lower portion of the housing into the test tube, the sperm separating filter can divide the test tube into an upper cavity and a lower cavity, and the sperm separating filter provides passage for the motile spermatozoa to swim upwardly from the lower cavity into the upper cavity.

The housing can be formed of crystal polystyrene or polycarbonate. The sperm separating filter can include a pore size greater than 5 µm and less than 30 µm. The sperm separating filter can be formed of polyethylene terephthalate or etched polycarbonate.

A motility-based spermatozoa separation apparatus is described which includes a hollow cylindrical body having an internal passageway. The hollow cylindrical body is configured for insertion into a test tube. A sperm separating member is positioned in the internal passageway. Upon insertion of the hollow cylindrical body into the test tube, the sperm separating member separates the test tube into a lower cavity and an upper cavity that is in fluid communication with the lower cavity. The sperm separating member provides passage for motile spermatozoa to swim upwardly from the lower cavity and into the upper cavity.

The sperm separating member can restrict the passage of non-motile spermatozoa therethough. The sperm separating member can be a filter. The filter can be one of a polyethylene terephthalate filter and an etched polycarbonate filter. The filter can include a pore size greater than 5 µm and less than 30 µm.

The hollow cylindrical body can extend between an upper portion and a lower portion, and the filter can be retained in the internal passageway with a retention ring.

In addition to human assistive reproductive technologies, the sperm separating teachings of the present application can be utilized in veterinary assisted reproductive technology including artificial insemination and in vitro fertilization (e.g., for bovine, equine, porcine, as well as other mammals).

Other embodiments include unique connection apparatuses, systems, and methods. Further embodiments, inventions, forms, objects, features, advantages, aspects, and benefits of the present application are otherwise set forth or become apparent from the description and drawings included herein.

### Brief Description of the Drawings

The description herein makes reference to the accompanying drawings wherein like reference numerals refer to like parts throughout the several views, and wherein:
Fig. 1 is an exploded view of an exemplary test tube insertable sperm motility-based separating device which is fashioned in accordance with the present principles;
Fig. 2 is a perspective view of the test tube insertable sperm motility-based separating device of Fig. 1, depicted in an assembled state;
Fig. 3 is a perspective view of a further exemplary test tube insertable sperm motility-based separating device which is fashioned in accordance with the present principles, the device shown in an unassembled state;
Fig. 4 is a perspective view of the test tube insertable sperm motility-based separating device of Fig. 3, the device depicted in an assembled state;
Fig. 5 is a perspective view of yet a further exemplary test tube insertable sperm motility-based separating device which is fashioned in accordance with the present principles, the device shown in an un-assembled state;
Fig. 6 is a perspective view of the test tube insertable sperm motility-based separating device of Fig. 5, depicted in an assembled state;
Fig. 7 is an illustrative view of another exemplary test tube insertable sperm motility-based separating device which is fashioned in accordance with the present principles, depicting a kit for separating motile sperm in a test tube, which includes a filter, unidirectional membrane, mechanical push device, and rubber seal, the unidirectional membrane and the filter allow motile sperm to swim through the pores and be collected at the top, and the mechanical push device allowing for a secure and controlled placement of the device, wherein the rubber seal holds the device in place;

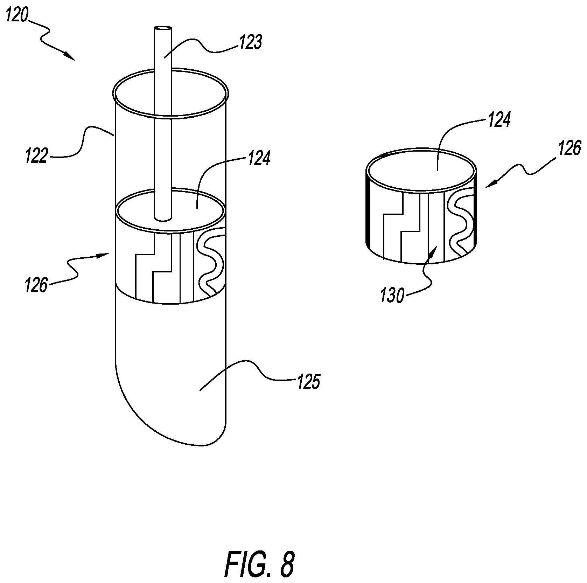

Fig. 8 is an illustrative view of yet a further exemplary test tube insertable sperm motility-based separating device which is fashioned in accordance with the present principles, depicting a kit for separating motile sperm in a test tube, utilizing a sperm separating device with microfluidic channels to sort and collect the sperm at the top of the microfluidic channels, a rod to set the separating device in place, and adhesive to retain the sperm separating device in place while inside the tube, the microfluidic channels being designed to collect only progressively motile sperm (the sperm that can travel in large arcs or straight lines, as opposed to tight circles, flailing, or no movement);
Fig. 9 is an illustrative view a further exemplary test tube insertable sperm motility-based separating device which is fashioned in accordance with the present principles, depicting a kit for separating motile sperm in a test tube, the kit including a filter with a rubber seal and a mucus-like gel designed to simulate cervical mucus (i.e., which acts as a natural sperm filter within the female reproductive tract);
Fig. 10A is an illustrative view of a yet a further exemplary test tube insertable sperm motility-based separating device which is fashioned in accordance with the present principles, depicting a kit for separating motile sperm in a test tube, the kit utilizing a filter and unidirectional barrier ice wherein silicone is used to create suction against the test tube to hold it in place along with a simple push rod to position the device, with a semen collection zone above the device;
Fig. 10B is an illustrative view of exemplary use of the exemplary test tube insertable sperm motility-based separating device of Fig. 10A;
Fig. 11A is an illustrative view of another exemplary test tube insertable sperm motility-based separating device which is fashioned in accordance with the present principles, depicting a kit for separating motile sperm in a test tube, utilizing a unidirectional barrier device and a chemotaxis attractant to separate and collect the separated or sorted sperm;
Fig. 11B is an illustrative top view of the unidirectional barrier device of the exemplary device of Fig. 11A;
Fig. 11C is an illustrative view of an external positioning device for the eighth device of Fig. 11A which holds the test tube, with a magnet and a rubber seal to hold the unidirectional barrier device in place;
Fig. 12A is an illustrative view of a ninth exemplary test tube insertable sperm motility-based separating device which is fashioned in accordance with the present principles, depicting a kit for separating motile sperm in a test tube, utilizing a sperm separation/sorting device having microfluidic channels and a rubber seal rather than an adhesive to hold the channels in place;
Fig. 12B is an illustrative view of an external positioning device useful for the ninth exemplary separating device of Fig. 12A that holds the test tube in place, with a magnetic ring to position the sperm separation sorting device within the test tube;
Fig. 13 is a perspective view of another exemplary form of a sperm separating device for insertion in a test tube fashioned in accordance with the present principles, the test tube sperm separator or sorting device shown assembled;
Fig. 14 is a top view of the test tube sperm separating device of Fig. 13;
Fig. 15 is a side view of the test tube sperm separating device of Fig. 13;
Fig. 16 is a perspective view of another exemplary embodiment of a sperm separating device for use in a test tube fashioned in accordance with the present principles, the test tube sperm separation device shown assembled but without a rubber seal;
Fig. 17 is a top view of the test tube sperm separating device of Fig. 16;
Fig. 18 is an enlarged portion of the honeycomb structure of the sperm separating device of Fig. 16;
Fig. 19 is a perspective view of the test tube sperm separating device of Fig. 16 with a rubber seal but without the honeycomb structure;
Fig. 20A is an enlarged view of an exemplary sperm separating member, being a non-woven filter membrane manufactured by Cytiva^{®} of etched polycarbonate, for use in the sperm separating devices described herein;
Fig. 20B is an enlarged view of another exemplary sperm separating member, being a woven filter membrane manufactured by pluriSelect ^{®} of PET, for use in the sperm separating devices described herein;
Fig. 20C is an enlarged view of yet another exemplary sperm separating member, being a woven filter membrane manufactured by Sterlitech^{®} of nylon, for use in the present test tube sperm separating devices described herein; and
Fig. 21 is a perspective view of another exemplary form of a test tube insertable motility-based sperm separating device fashioned in accordance with the present principles, which includes a hollow cylindrical housing having a sperm separating member located at a lower portion, this being a preferred form due to simplicity of use and manufacture;
Fig. 22A is a side hidden line view of the sperm separating device of Fig. 21, depicting a passageway extending between open ends of the housing, with a filter being retained in the passageway at a lower portion of the housing;
Fig. 22B is a cross-sectional enlarged partial view directed toward the lower portion of the sperm separating device of Fig. 22A, which depicts the filter being retained in the lower portion by a filter retention member;
Fig. 23 is a perspective view of the filter retention member of Fig. 22B;
Fig. 24 is a perspective view of an exemplary sperm separating member, which is depicted as a woven filter disk;
Fig. 25 is a perspective view of the sperm separating device of Fig. 1 and also depicting an exemplary 15 mL conical test tube in which the sperm separating device can be inserted for use;
Fig. 26 is a perspective view of the sperm separating device of Fig. 25, with the housing inserted in the test tube depicting an in-use configuration;
Fig. 27 is a perspective view of another exemplary form of a test tube insertable motility-based sperm separating device fashioned in accordance with the present principles, which includes a hollow cylindrical housing located in a test tube similar to Fig. 26, but which includes a frusto-conical tip having a filter located therein for ease of use with smaller sperm samples (e.g., .25 mL frozen sperm specimens);
Fig. 28 is a side perspective view of another exemplary form of a test tube insertable motility-based sperm separating device fashioned in accordance with the present principles, the device being similar to the sperm separating device of Fig. 1 except that the conical test tube is larger (e.g., 50 mL as may be utilized for porcine semen samples) and the sperm separating device is enlarged to be closely engaged therein;
Figs. 29A-29D are illustrative views of the test tube insertable motility-based sperm separating device of Fig. 28, depicting progressive view of use of the device (e.g., separating motile sperm from a semen sample);
Fig. 30 depicts another exemplary form of a test tube insertable motility-based sperm separating device, fashioned in accordance with the present principles, and illustrating a filter housing having positioning members extending upwardly therefrom; and
Fig. 31 depicts the sperm separating device of Fig. 30, inserted within a test tube and ready for use.

The accompanying drawings incorporated in and forming a part of the specification illustrate various forms and features of the present application; however, the present application should not be construed as being limited to those specific embodiments depicted in the drawings.

### Detailed Description

For purposes of promoting an understanding of the principles of the invention, reference will now be made to the embodiments illustrated in the drawings and specific language will be used to describe the same. It will nevertheless be understood that no limitation of the scope of the invention is thereby intended, any alterations and further modifications in the illustrated device, and any further applications of the principles of the invention as illustrated therein being contemplated as would normally occur to one skilled in the art to which the invention relates.

As utilized herein, the terms "sperm" and "spermatozoa" refer to the male gametes involved in sexual reproduction and are to be viewed as interchangeable (e.g., sperm being a shortened term for spermatozoa). The term "sperm separating member" is intended to include filters (e.g., foamed membranes, woven/mesh filters, etched polymers, non-woven membranes, filter media), members having microfluidic channels, and other physical barrier-type members that provide passage for motile sperm to swim upwardly therethrough, while restricting the passage of non-motile sperm.

Referring generally to the Figures, there are shown devices, apparatuses, and systems for swim-up motility-based separation of spermatozoa within a test tube. The devices, systems, apparatuses, and methods described herein enable sperm with high motility to be obtained from a semen sample, absent the use of centrifugation and detrimental effects thereof.

The devices, systems, apparatuses, and methods of the present application can be utilized to separate and prepare progressively motile sperm for assistive reproductive technologies, which include artificial insemination, in-vitro fertilization (IVF), etc. The teachings of the present application can be utilized to separate human or animal (e.g., bovine, equine, swine, etc.) sperm. However, as will be appreciated to a person of skill in the art, the sperm separating member (e.g., filter, filtering medium, microfluidic channels, etc.) passage and/or pore size can be altered depending upon the specific species of sperm to be separated (e.g., larger pores may be utilized for species having larger spermatozoa cells).

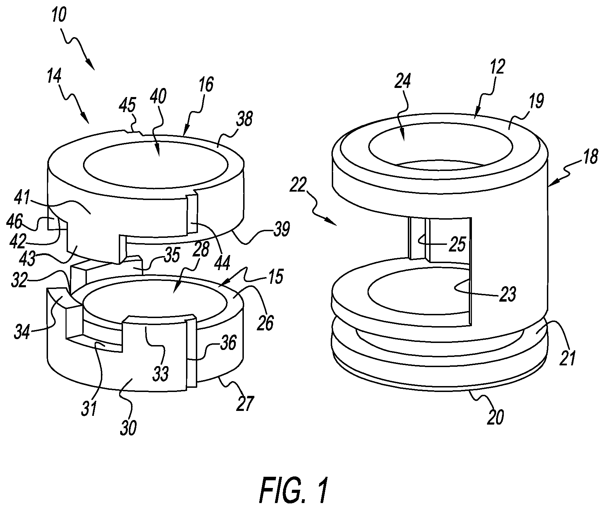

Fig. 1 provides an illustration of an exemplary embodiment or version of sperm separating motile sperm within a test tube, generally designated 10. The test tube sperm swim-up separator or sorting device 10 is formed of an appropriate material such as plastic or metal and is fashioned to hold a filter, filter/filtering medium, or the like (not shown) that allows sperm to be separated or sorted by motility.

The test tube sperm swim-up separator or sorting device 10 has a first part or base 12 and a second part or insert 14 that is formed of a first or lower piece 15 and a second or upper piece 16. The nomenclature first and second is arbitrary here and throughout unless specifically indicated otherwise. The insert 14 is configured to receive a sperm filter or filtering medium (not shown) and be received in the base 12. The base 12 is defined by a cylindrical body 18 having a generally planar top 19 and a generally planar bottom 20, with a generally interior cylindrical cavity 22. The cylindrical body 18 is sized for reception in a test tube such as, but not necessarily, a 15mL test tube. The top 19 has a generally circular opening 24 that provides communication with the interior cavity 22. The cylindrical sidewall of the cylindrical body 18 has an opening 23 that extends approximately half-way around the cylindrical sidewall. The inside cylindrical sidewall has a vertical notch 25 at one end of the notch 23, and a vertical notch (not seen) on the other end of the notch 23. A circumferential groove 21 is provided proximate the bottom 20 that is configured to receive a circular rubber or similar seal such as an O-ring or the like (not shown). The seal allows the device 10 to be positioned and held within a test tube at a desired location and to block semen flow from around the device 10.

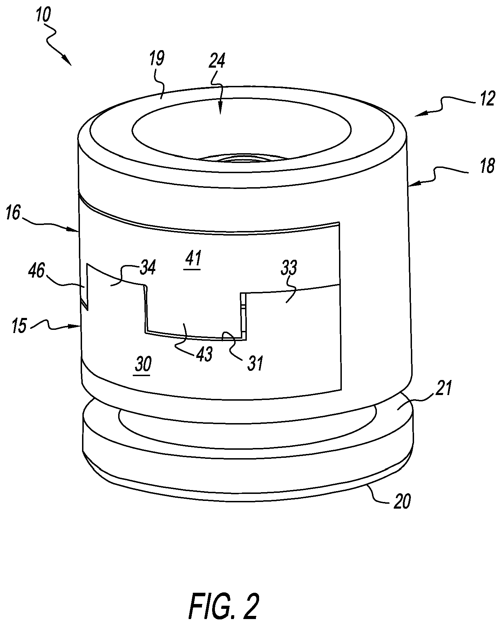

The insert 14 is sized and configured to be received in the interior cavity 22 of the base 12 via the notch 23 (see Fig. 2 for a view of the device 10 assembled). As such, the lower piece 15 and the upper piece 16 are generally cylindrical. The lower piece 15 defines a top 26 and a bottom 27 with a generally tubular hole 28 that extends from the top 26 to the bottom 27. Situated on the outer circular sidewall of the lower piece 15, is a wall 30 that extends radially outwardly from the outer circular sidewall and approximately one-half of the circumference of the outer circular sidewall. The wall 30 has a first end defining a vertical cutout 36 configured to abut the vertical notch (not shown) of the inside cylindrical sidewall of the base 12 that is opposite the vertical notch 25 shown when the insert 14 is received in the base 12. The wall 30 has a second end defining a vertical cutout (not seen) but like vertical cutout 36 configured to abut the vertical notch 25 of the inside cylindrical sidewall of the base 12 when the insert 14 is received in the base 12. The wall 30 has a first extension 33 radially adjacent the vertical cutout 36 that rises above the top 26 of the lower piece 15, a first squared notch 31 radially adjacent the first extension 33, a second extension 34 radially adjacent the first squared notch 31 radially opposite to the first extension 33 that rises above the top 26 of the lower piece 15, a second squared notch 32 radially adjacent the second extension 34 radially opposite the second extension 34, and a third extension 35 radially adjacent the second squared notch 32 radially opposite the second extension 34 that rises above the top 26 of the lower piece 15.

The upper piece 16 defines a top 38 and a bottom 39 with a generally tubular opening 40 that extends from the top 38 to the bottom 39. Situated on the outer circular sidewall of the upper piece 16, is a wall 41 that extends radially outwardly from the outer circular sidewall and approximately one-half of the circumference of the outer circular sidewall. The wall 41 has a first end defining a vertical cutout 44 configured to abut the vertical notch (not shown) of the inside cylindrical sidewall of the base 12 that is opposite the vertical notch 25 shown when the insert 14 is situated in the base 12. The wall 41 has a second end defining a vertical cutout 45 like vertical cutout 44 configured to abut the vertical notch 25 of the inside cylindrical sidewall of the base 12 when the insert 14 is received in the base 12. The wall 30 has a first extension 43 radially proximate the vertical cutout 44 that rises below the bottom 39 of the upper piece 16, a first squared notch 42 radially adjacent the first extension 43, a second extension 46 radially adjacent the first squared notch 42 radially opposite to the first extension 43, a second squared notch (not shown) radially adjacent the second extension 46 radially opposite the second extension 46.

The upper piece 16 and the lower piece 15 fit together to hold a filter, filter medium, or the like (not shown) then retained in the base 12 as seen in Fig. 2. When assembled, the extensions of the lower piece 15 fit into the notches of the upper piece 16, while the extensions of the upper piece 16 fit into the notches of the lower piece 15. With the addition of a rubber seal (not shown) in the circumferential groove 21 and a filter, filter medium or the like (not shown), the device 10 is ready for insertion into a test tube for sperm separation.

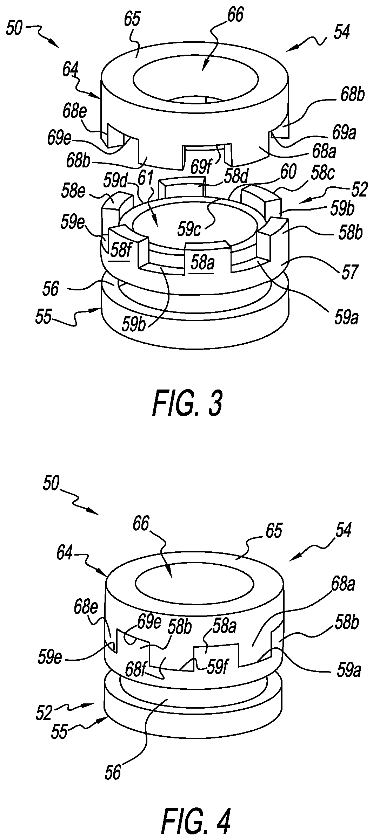

Figs. 3-4 provide an illustration of another exemplary embodiment or version of a device for sperm swim-up separation or sorting of motile sperm within a test tube, generally designated 50. The test tube sperm swim-up separator or sorting device 50 is formed of an appropriate material such as plastic or metal and is fashioned to hold a filter, filter/filtering medium, or the like (not shown) that allows sperm to be separated or sorted by motility. The test tube sperm swim-up separator or sorting device 50 is formed of two (2) parts or pieces, a lower part, piece, or base 52 and an upper part or piece 54.

The base 52 has a generally cylindrical body 55 with a circumferential groove or channel 56 proximate the bottom of the cylindrical body 55 configured to receive a circular rubber or similar seal such as an O-ring or the like (not shown). The seal allows the device 50 to be positioned and held within a test tube at a desired location and to block semen flow from around the device 50. The cylindrical body 55 defines a top 60 with a generally circular hole defining a cylindrical cavity 61 that extends through the cylindrical body 55 to allow sperm to travel through the cylindrical body 55. The cylindrical cavity 61 may also hold a filter, filter medium, or the like (not shown). The upper portion of the cylindrical body 55 includes six (6) notches 59a-f spaced about the circumference of the upper portion of the cylindrical body 55 forming six (6) extensions 58a-f spaced about the circumference of the upper portion of the cylindrical body 55. Each extension 58a-f extends above the top 60 of the cylindrical body 55.

The upper part 54 is defined by a generally cylindrical body 64 defining a top 65 and a generally circular opening into a cylindrical cavity 66 that extends through the cylindrical body 64 to allow sperm to travel through the cylindrical body 64. The cylindrical body 64 is sized in like manner to the cylindrical body 55 of the base 52. Six (6) notches 69a-f are provided in the cylindrical body 64 spaced about the circumference thereof forming six (6) extensions 68a-f spaced about the circumference of the lower portion of the cylindrical body 55. Each extension 68a-f extends below the bottom of the cylindrical body 64. The extensions 68a-f of the cylindrical body 64 of the upper part 54 are sized and configured for reception in the notches 59a-f of the cylindrical body 55 of the base 52, while the extensions 58a-f of the cylindrical body 55 of the base 52 are sized and configured for reception in the notches 69a-f of the cylindrical body 64 of the upper part 54. This is shown in Fig. 4 where the two parts 52, 54 are combined. With the addition of a rubber seal (not shown) in the circumferential groove 56 and a filter, filter medium or the like (not shown), the device 50 is ready for insertion into a test tube for sperm separation.

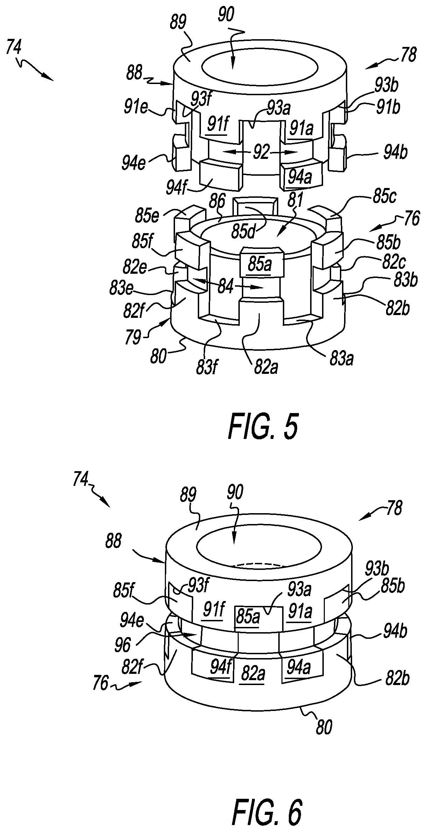

Figs. 5-6 provide an illustration of another exemplary embodiment or version of a device for swim-up separation or sorting of motile sperm within a test tube, generally designated 74. The test tube sperm swim-up separator or sorting device 74 is formed of an appropriate material such as plastic or metal and is fashioned to hold a filter, filter/filtering medium, or the like (not shown) that allows sperm to be separated or sorted by motility. The test tube sperm swim-up separator or sorting device 74 is formed of two (2) parts or pieces, a lower part, piece, or base 76 and an upper part or piece 78.

The base 76 has a generally cylindrical body 79 defining a bottom 80 and a top 86 with a circumferential groove or channel 84 approximately midway between the bottom 80 and the top 86 that is configured to receive a circular rubber or similar seal such as an O-ring or the like (not shown). The seal allows the device 76 to be positioned and held within a test tube at a desired location and to block semen flow from around the device 76. The cylindrical body 79 has a generally circular hole opening into a cylindrical cavity 81 that extends through the cylindrical body 79 to allow sperm to travel through the cylindrical body 79. The cylindrical cavity 81 may also hold a filter, filter medium, or the like (not shown). The cylindrical body 79 includes six (6) notches 83a-f spaced about the circumference of the cylindrical body 79. The six (6) notches 83a-f and the circumferential groove 84 form six (6) extensions 85a-f spaced about the circumference of the upper portion of the cylindrical body 79 with each extension 85a-f extending above the top 86 of the cylindrical body 79, and six (6) steps 82a-f situated axially below the six (6) extensions 85a-f, respectively.

The upper part 78 is defined by a generally cylindrical body 88 defining a top 89 and a generally circular opening into a cylindrical cavity 90 that extends through the cylindrical body 88 to allow sperm to travel through the cylindrical body 88. The cylindrical body 88 is sized in like manner to the cylindrical body 79 of the base 76. Six (6) notches 93a-f are provided in the cylindrical body 88 spaced about the circumference thereof forming six (6) extensions 94a-f spaced about the circumference of the lower portion of the cylindrical body 88. Each extension 94a-f extends below the bottom of the cylindrical body 88. A circumferential groove or channel 92 is provided in the cylindrical body 88 approximately midway between the bottom and the top 89 that is configured to receive the circular rubber or similar seal such as an O-ring or the like (not shown) of the base 76. The six (6) notches 93a-f and the circumferential groove 92 form six (6) extensions 94a-f spaced about the circumference of the lower portion of the cylindrical body 88 with each extension 93a-f extending below the bottom of the cylindrical body 88, and six (6) steps 91a-f situated axially above the six (6) extensions 64a-f, respectively. The extensions 94a-f of the cylindrical body 88 of the upper part 78 and the steps 91a-f are sized and configured for reception in the notches 83a-f of the cylindrical body 79 of the base 76, while the extensions 85a-f and the steps 82a-f of the cylindrical body 79 of the base 76 are sized and configured for reception in the notches 93a-f of the cylindrical body 88 of the upper part 78. This is shown in Fig. 6 where the two parts 76, 78 are combined. With the addition of a rubber seal (not shown) in the circumferential groove 84 and a filter, filter medium or the like (not shown), the device 74 is ready for insertion into a test tube for sperm separation.

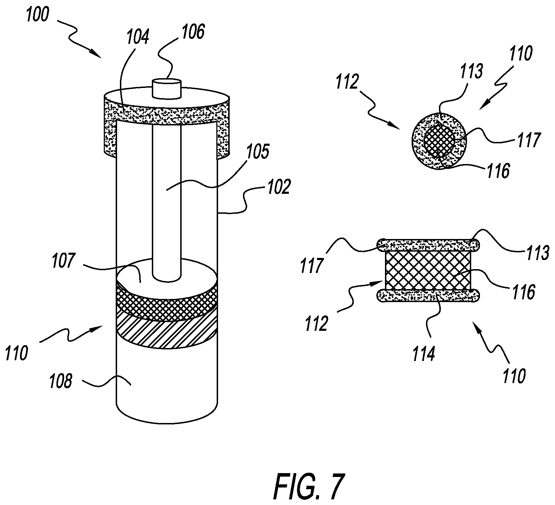

Fig. 7 illustrates an exemplary method, apparatus and devices (kit) for sperm separation or sorting within a test tube, generally designated 100, fashioned in accordance with the present principles, employing a filter device, a unidirectional membrane, a mechanical push device, and a rubber seal on the filter device to hold the filter device in a test tube. The unidirectional membrane combined with the filter allow the sperm to swim through the pores of the unidirectional membrane and be collected at the top of a test tube in which has the sperm and unidirectional filter. The mechanical push device allows for a secure and controlled placement of the device. The rubber seal holds the device in place. The method 100 includes using a test tube 102 such as, but not necessarily, a 15mL test tube. A device 110 for separating, sorting, or filtering sperm from a semen (sperm) sample 108 in the test tube 102 is shown. The separating, sorting, or filtering device 110 represents any of the sperm separation/sorting devices described herein, including the separating, sorting, or filtering device 110 shown in Fig. 7. The separating, sorting, or filtering device 110 is generally cylindrical to fit into the test tube 102 and is defined by a body 112 with a top 113 and a bottom 114 holding a filter and unidirectional membrane 116 therebetween. The rubber seal 117 is provided about the top 113.

The mechanical push device for placing the filtering device 110 into the test tube 102 includes a cover 104 that is configured to over the opening of the test tube 102 along with side brackets to help position and stabilize the cover in place. The mechanical push device includes a disc 107 attached to a rod 105 that extends through a hole in the cover 104 and terminates in a cap or handle 106. The disc 107, rod 105, and cap 106 are axially movable within the test tube 102 and relative to the cover 104 for positioning the filtering device 110 within the test tube 102 and/or relative to the sperm sample 108. The disc 107 is sized in like fashion as the top 113 of the device body 112 to properly seat the filtering device 110 in the test tube 102. Fig. 7 depicts the mechanical push device having appropriately positioned the filtering device 110 in the test tube 102. The cap or handle 108 can then be used to pull the disc 107 axially away from the filtering device 110 via the rod 105 which is attached to the disc 107.

Fig. 8 illustrates another method, apparatus and devices (kit) for sperm separation or sorting within a test tube, generally designated 120, fashioned in accordance with the present principles, employing a sperm filtering device 126 providing microfluidic channels 130 to sort and enable collection of sperm at the top of the channels 130, a rod 123 to set the sperm filtering device 126 in place via a disc attachment 124 coupled to the filtering device 126, and adhesive to keep the filtering device 126 in place while inside the test tube 122. The channels 130 are designed to collect only progressively motile sperm (the sperm that can travel in large arcs or straight lines, as opposed to tight circles or no movement) from a sperm sample 125 in the test tube 122.

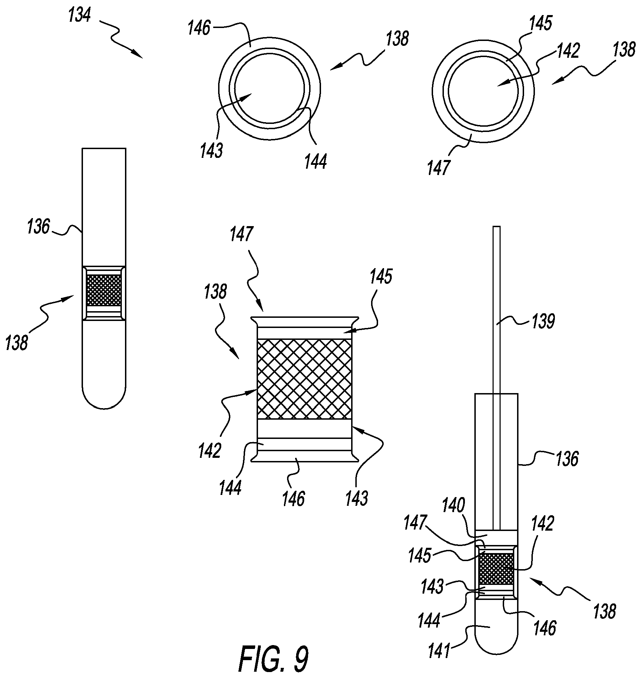

Fig. 9 illustrates another method, apparatus and devices (kit) for sperm separation or sorting within a test tube, generally designated 134, fashioned in accordance with the present principles, similar to the kit 100 of Fig. 7, employing a sperm sorting device 138 combining two of sperm-sorting concepts by using a sperm filtering medium 142 and a mucus-like gel 143 designed to simulate cervical mucus, acting as a natural sperm filter within the female reproductive tract to provide a membrane encapsulated, and a mechanical push device. This provides extra filtration, improving sensitivity of sperm filtration. The sperm filtering medium 142 and mucus-like gel 143 of the sperm sorting device 138 is retained by a lower stabilizing ring 144 and an upper stabilizing ring 145. A lower seal 146 is provided around the lower stabilizing ring 144 while an upper seal 147 is provided around the upper stabilizing ring 145. The mechanical push device is used to place and position the sperm sorting device 138 in the test tube 136. The test tube 136 shown on the right side of Fig. 9, holds a sperm sample 141, while the mechanical push device, consisting of a disc 140 attached to a rod 139, is shown having placed the sperm sorting device 138 appropriately in the test tube 136.

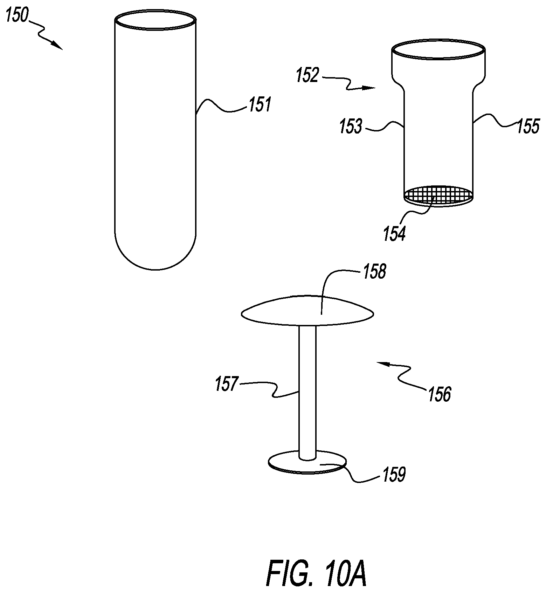

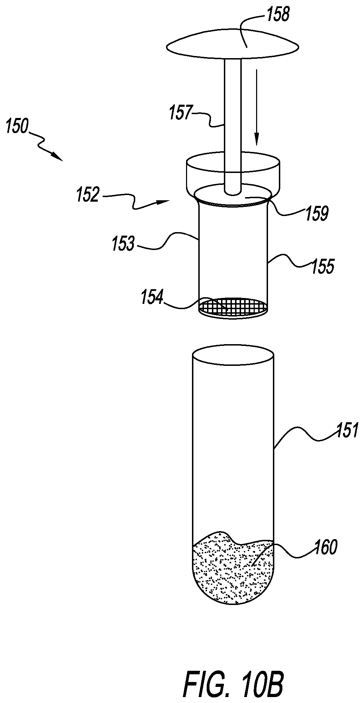

Figs. 10A-B illustrates another method, apparatus and devices (kit) for sperm separation or sorting within a test tube, generally designated 150, fashioned in accordance with the present principles, employing a sperm sorting device 152 having a filter and unidirectional barrier 154 within a silicone body 153 shaped to create suction against a test tube 151 to hold the body 153 in place, along with a simple push rod device 156 to position the sperm sorting device 152. The body 153 also has a semen collection zone/area 155 above the filter and unidirectional barrier 154. The push rod device 156 includes a handle or head 158 attached to a rod 157 having a disc 159 to abut and push against the sperm sporting device 152 when inserting it into the test tube. Fig. 10B illustrates the insertion process.

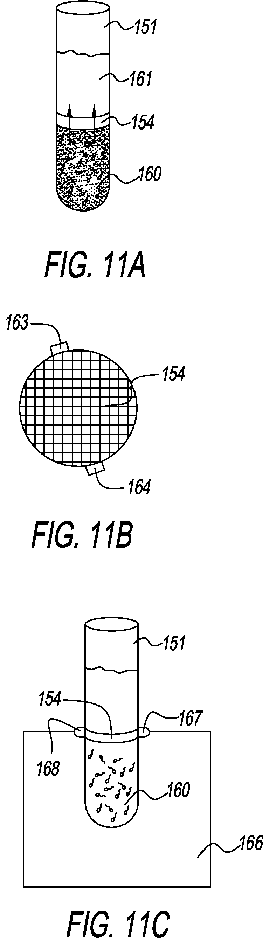

Figs.11A-C illustrates another method, apparatus and devices (kit) for sperm separation or sorting within a test tube, fashioned in accordance with the present principles for use with a test tube 151. A sperm sorting device employs a unidirectional barrier 154 and a chemotaxis attractant 161 to separate and collect healthy sperm from a sperm sample 160. Magnets 163, 164 are provided on the device that act with magnets 167, 168 of an external positioning device 166 to hold the device in place. A rubber seal (not seen) may also be used. The magnets 163, 164 and magnets 167, 168 may be a single magnet or more than two.

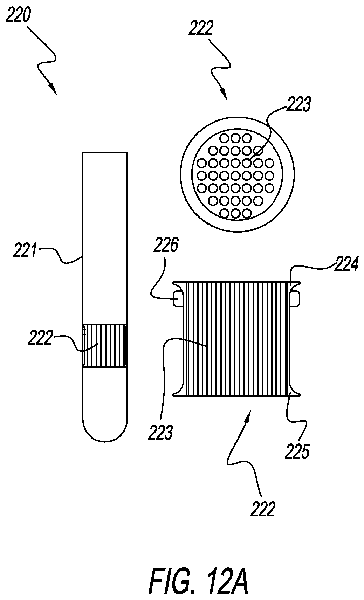

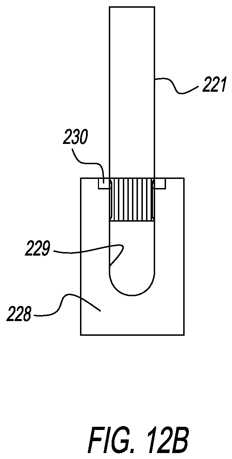

Figs. 12A-B illustrate another method, apparatus and devices (kit) for sperm separation or sorting within a test tube 221, generally designated 220, fashioned in accordance with the present principles, that employs a sperm separating device 222 with microfluidic channels 223 for sorting, with a rubber seal 224 to hold the sperm separating device 222 in place within the test tube 221. The sperm separating device 222 also includes a magnetic ring 226 for holding and positioning the sperm separating device 222 by an external mechanism 228, which itself utilizes a magnetic ring 230 to act with the magnetic ring 226 of the sperm separating device 222 and position the sperm separating device 222 within the test tube 221.

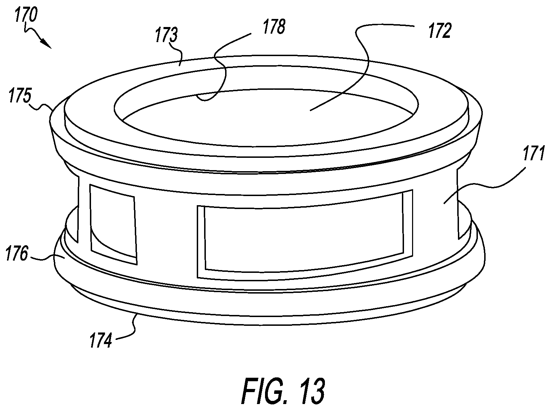

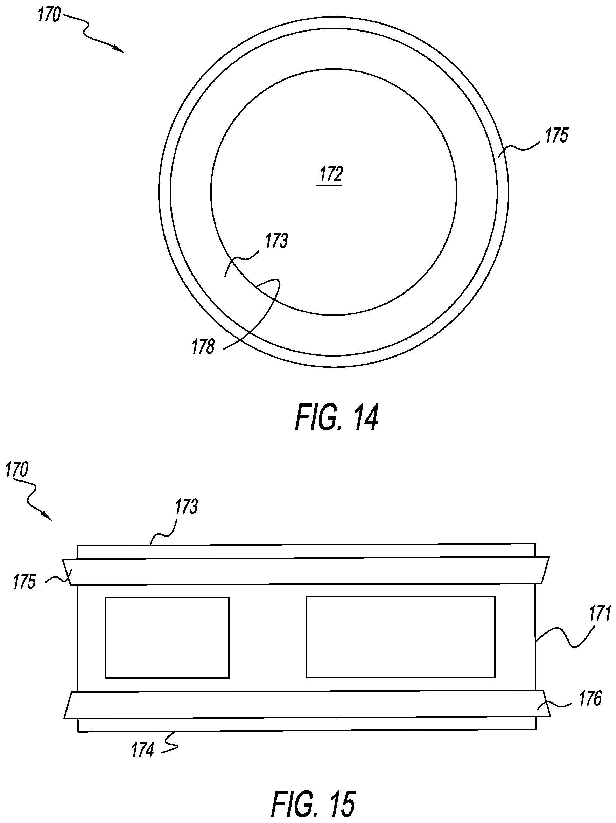

Figs. 13-15 illustrate another device for sperm separation or sorting, generally designated 170, within a test tube, fashioned in accordance with the present principles for use with the present methods. The sperm separating device 170 is defined by a generally cylindrical housing or body 171 formed of a suitable material that is preferably, but not necessarily, 3-D printed and which can withstand sterilization. The housing 171 is also preferably sized to fit inside a test tube, the test tube preferably, but not necessarily, being of typical dimensions (e.g. a 15 mL tube). The housing 171 defines a top 173 with an opening 178 and a bottom 174 with an opening (not seen) with an internal cavity in communication with the top and bottom openings. The housing 171 holds a cylindrical filter, filter medium 172 or the like that is about 5mm tall. An upper rubber or silicone seal 175 is provided proximate the top 173, while a lower rubber or silicone seal 176 is provided proximate the bottom 174. The upper and lower seals 175, 176 are designed to hold the device/filter in place within the test tube.

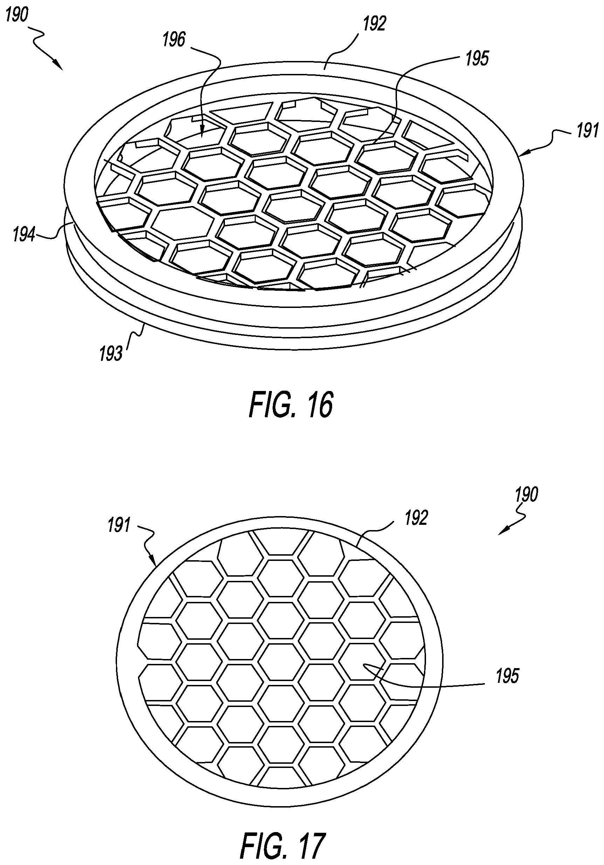

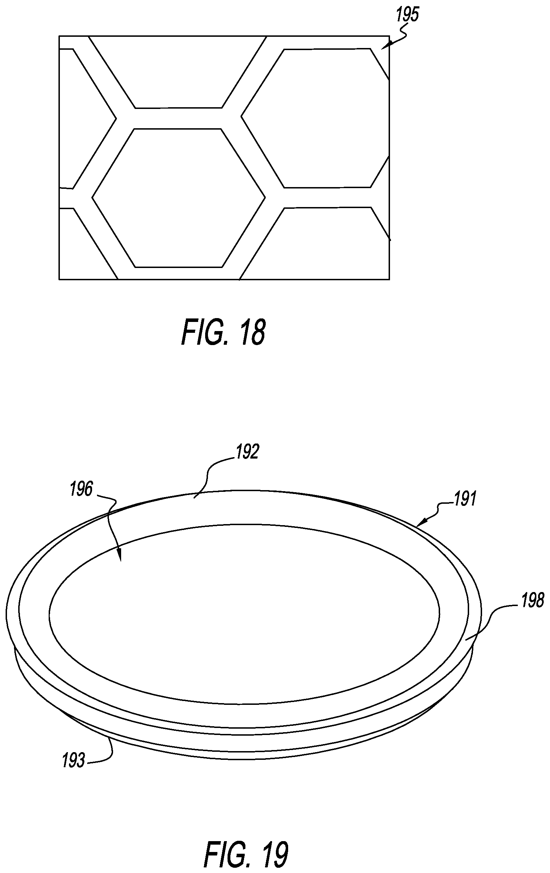

Figs. 16-19 illustrate another device for sperm separation or sorting, generally designated 190 fashioned in accordance with the present principles for use with the present methods. The sperm separating device 190 is defined by a generally disc-shaped housing or body 191 formed of a suitable material that is preferably, but not necessarily, 3-D printed and which can withstand sterilization. The housing 191 is also preferably sized to fit inside a test tube, the test tube preferably, but not necessarily, being of typical dimensions (e.g. a 15 mL tube). The housing 191 defines a top 192 with and a bottom 193 with a disc-shaped internal cavity. The housing 191 has a circumferential groove 194 configured to hold a silicone, rubber, or similar seal 198 (see Fig. 19). The inside of the cavity of the housing 191 is a slit configured to hold a flat disc sperm separation/sorting filter/membrane 196 (see Fig. 9), similar in thickness to a coffee filter. A honeycomb support structure, lattice, network or the like 195 also preferably, but not necessarily, 3-D printed and able to withstand sterilization. The purpose of the honeycomb structure 195 is to ensure that the filter/membrane 196 stays in place during the use of the device by preventing damage and by providing extra support for the filter/membrane 196. With the honeycomb structure 195, if a pipette does run into the filter/membrane 196 during collection, it is less likely to puncture all the way through and reduces the risk of a failed collection. The honeycomb structure 195 maximizes the available surface area of the filter/membrane while still providing a lattice structure to protect the filter/membrane. Fig. 19 shows a top view of the device 190 complete with the filter/membrane 196 and the silicone/rubber seal 198. The honeycomb sits just below the filter/membrane.

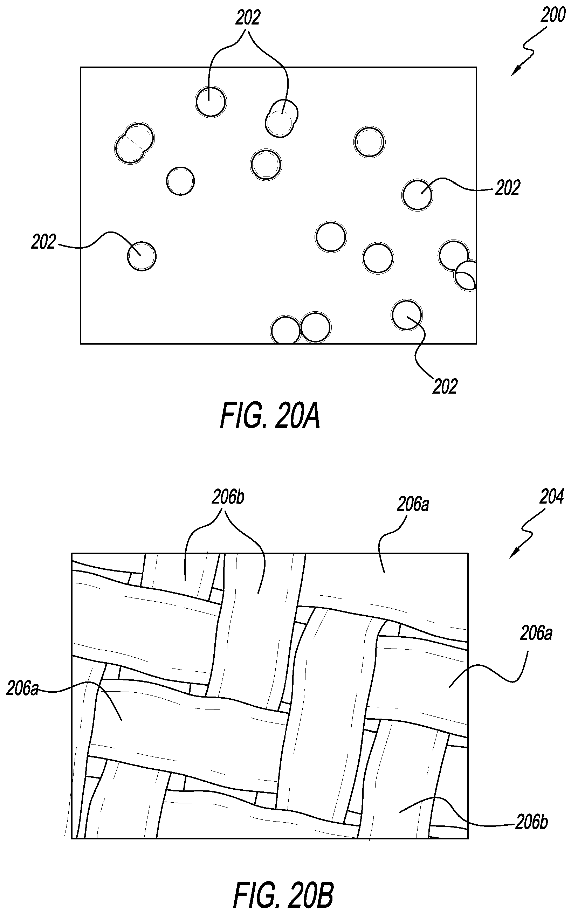

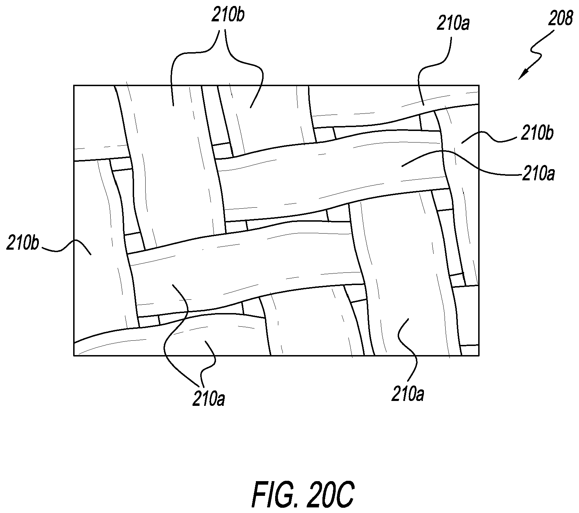

Figs. 20A-C provide examples of exemplary types of filter/membrane material that may be used with the present devices for sperm swim-up separation or sorting of motile sperm within a test tube. Fig. 20A is a close-up of an exemplary filter/membrane material 200 being a microchannel filter having a plurality of angled channels 202. Fig. 20B is a close-up of another exemplary filter/membrane material 204 having a weave of a plurality of first bands 206a of a first direction, and a plurality of second bands 206b of a second direction that is generally perpendicular to the first direction to create the weave. Fig. 20C is a close-up of another exemplary filter/membrane material 208 having a weave of a plurality of first bands 210a of a first direction, and a plurality of second bands 210b of a second direction that is generally perpendicular to the first direction to create the weave.

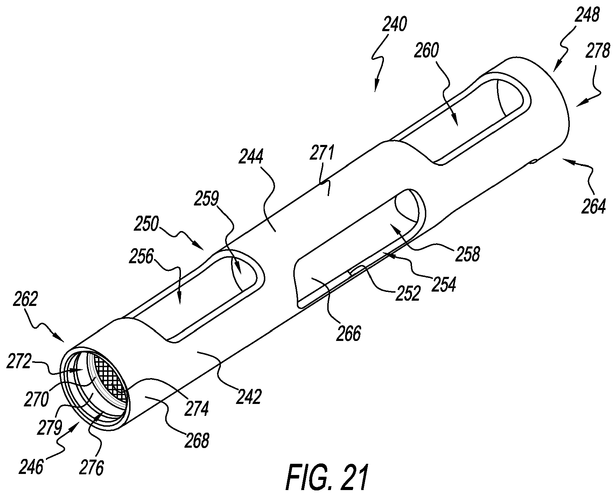

Referring now generally to Figs. 21-26, a further form of a sperm separating device 240 will now be described. The sperm separating device 240 is a preferred form of the present application. The sperm separating device 240 includes a body 242 which is configured for insertion into a test tube 320. The body 242 is depicted as housing 244. A sperm separating member 274 is housed in an internal area 272 of the housing 244. The housing 244 is depicted as having a hollow cylindrical form 250 (e.g., a cylindrical shell-like form defined around longitudinal axis 290).

Referring now to Fig. 21, the hollow cylindrical form 250 includes an outer wall 254, which is depicted as defining a circumference of the housing 244. The outer wall 254 includes a thickness 252. The housing 244 extends between a first end 246 and a second end 248.

An internal passageway 266 extends between an opening 276 at the first end 246 and an opening 278 at the second end 248. The sperm separating member 274 is disposed in the internal passageway 266. The sperm separating member 274 is depicted as being positioned in the internal passageway 266 at the lower portion 262 of the housing 244, upwardly from the first end 246. The housing 244 includes an upper portion 264, which is located upwardly from the sperm separating member 274.

Referring now to Figs. 21-25, the outer wall 254 of the housing 244 can include a plurality of openings 256, 257, 258, 259, 260, and 261, which serve as viewing windows (e.g., enabling a user to easily see into the internal passageway 266). Upper openings 260 and 261 are positioned in the upper portion 264 of the housing 244, at diametrically opposite positions. Central openings 258, 259 are positioned at a central portion 271 of the housing 244, at diametrically opposite positions. Lower openings 256 and 257 are positioned in the outer wall 254 above the sperm separating member 274, toward the lower portion 262, and are also diametrically opposed. The openings 256, 257, 258, 259, 260, and 261 can include a rounded rectangular form 292.

A filter retention ring 279 can be utilized to retain the sperm separating member 274 in the internal passageway 266. The sperm separating member 274 can be positioned within a filter housing 270 (e.g., with the filter housing 270 encompassing a circumference of the separating member 274). The filter retention ring 279 retains the sperm separating member 274 by engaging with the filter housing 270 and the outer wall 254, as shown in Fig. 21.

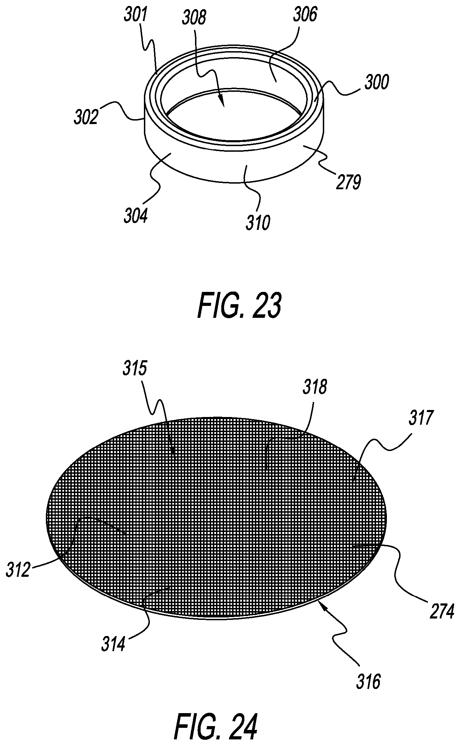

Preferably, the sperm separating member 274 can take the form of a filter 315, as shown in Fig. 23. The filter 315 includes a disc-like form extending between an upper surface 318 and a lower surface 316. The filter 315 is depicted as including a woven construction 312 having numerous pores 314 which are sufficiently sized for motile sperm to swim therethrough during use (e.g., swim-up motility-based sperm separation).

It is believed that for human, bovine, and porcine sperm, pores 314 sized greater than 5 µm and less than 30 µm can provide adequate sperm separation (i.e., with progressively motile sperm being able to swim upwardly toward the lower surface 316 of the filter 315, swimming through the pores 314 of the filter 315, and exiting the filter 315 at the upper surface 318). The filter 315 construction 312 and pores 314 are configured to restrict the passage of non-progressively motile sperm and non-motile sperm (e.g., with the non-progressively motile sperm and non-motile sperm either being unable to enter the pores 314 or being retained within the pores 314).

It is believed that for sperm of most species, the sizing of pores 314 may fall in the range of 5 µm to 100 µm. However, as will be appreciated to a person of skill in the art, sperm of different species may require different pore 314 sizing for adequate swim-up, motility-based separation (e.g., as sperm of different species can include different sizing and structural characteristics).

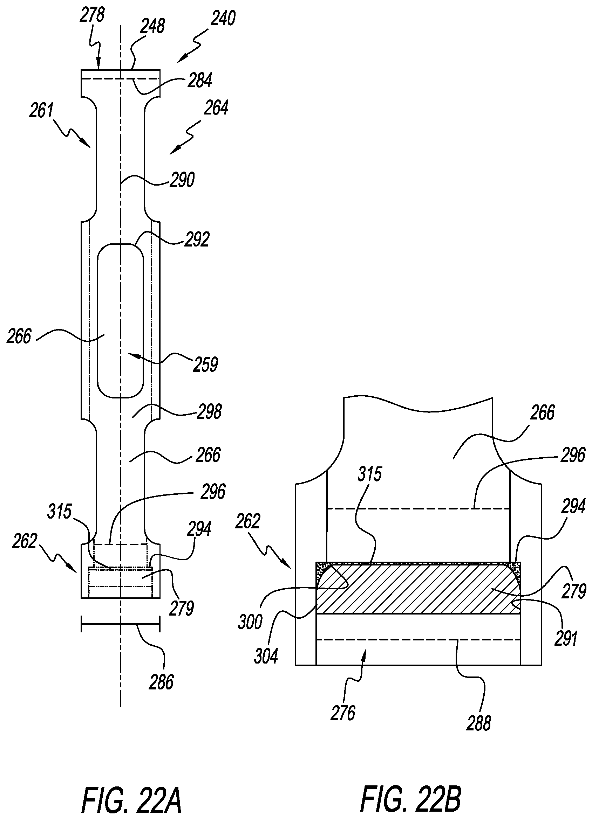

Referring now to Figs. 22A-23, the filter 315 can be directly retained in the internal passageway 266 by the filter retention ring 279 (e.g., the filter 274 being sandwiched between the filter retention ring 279 and a filter retention ledge 294 of the body 242). The filter retention ring 279 has a body 302 that includes ring-like shape 310. The filter retention ring 279 includes an outer surface 304 and an inner surface 306. A central opening 308 extends through the body 302 and is surrounded by the inner surface 306. An upper rim 300 of the filter retention ring 279 can be beveled 301.

The lower portion 262 of the housing 244 is configured to be closesly received by a test tube when inserted therein. The housing 255 can include a reduction in outer diameter between the upper portion 264 and the lower portion 264, which can ease insertion of the housing 244 into the test tube and providing for close engagement between the housing 244 and the test tube when inserted therein. The outer diameter 286 at the lower portion 262 is depicted as being around ten (10) percent smaller than the outer diameter 284 at the upper portion 264. However, the dimensions of the sperm separating device 240 and relative sizing of components can be varied significantly depending upon the specific test tube to be inserted in.

As is best illustrated in Fig. 22B, the filter retention ledge 294 inwardly extends between an inner surface 291 of the lower opening 276 and the inner passageway 266 (directly above the filter above the filter 315). The diameter 296 of the inner passageway 266 above the filter 315 is smaller than the diameter 288 of the lower opening 276. The filter 315 is fixedly retained between the upper rim 300 of the filter retention ring 279 and the filter retention ledge 294. The outer surface 304 of the filter retention ring 279 firmly engages with the inner surface 291 in an interference fit.

The housing 244 is depicted as being formed of a polymer material 298, which can take the form of crystal polystyrene or polypropylene. The polymer material 298 is preferably transparent to provide a user the ability to easily view the central passageway 266 (e.g., to ease manipulation of a pipette for media insertion and removal of motile sperm from the central passageway 266 above the filter 315 after the motile sperm have swam upwardly therethrough). However, depending upon the specific application and desired characteristics of the housing 244, it is contemplated that the housing 244 can be formed of a variety of materials that do not detrimentally impact sperm.

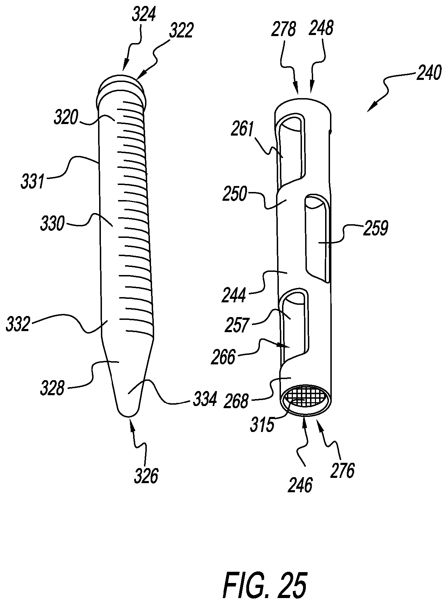

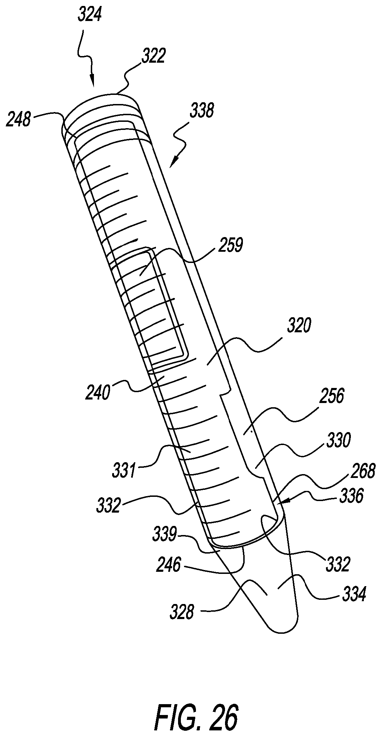

Referring now to Figs. 25 and 26, the lower portion 262 of the housing 244 is sized and shaped for insertion into a test tube 320. The test tube 320 is depicted as a 15 mL conical tube as is commonly utilized in human IVF laboratories. When the housing 244 is inserted into the 15 mL test tube, the filter 315 can be located near the 2 mL mark, while the housing 244 may extend down to around the 1.5 mL mark. This configuration is believed to provide adequate room for the typical human sperm sample size, while not requiring excess media and/or excess dilution of the motile sperm with media during removal from the upper cavity 33 (e.g., after the motile sperm have swam upwardly through the filter 315).

The test tube 320 includes an opening 324 at a first end 322. A second closed end 326 includes a conical form 328. The test tube includes a hollow cylindrical wall 331 having an inner surface 332. An upper cavity 330 is defined inside the hollow cylindrical wall 331. The test tube 320 includes a lower cavity 334 in which a semen sample will be placed prior to insertion of the sperm separating device 240.

The lower portion 262 of the sperm separating device 240 is inserted through the opening 325 in the test tube 320. The lower portion 262 is slid downwardly through the upper cavity 330 until the first end 246 engages the inner surface 332 at or near the conical transition 339 in the test tube 320. In this fully inserted configuration 338, the filter 315 is positioned above the lower cavity 334, containing the semen sample to be separated. The filter 315 is located below the upper cavity 330. In this manner, the filter 315 serves as a partition between the semen sample (e.g., including motile and non-motile sperm) in the lower cavity 344 and the upper cavity 330.

In this fully inserted configuration 338, the outer surface 268 of the housing 244 closely engages the inner surface 332 of the test tube 320 at or near the conical transition 339. The close engagement and contact of the lower portion 262 of the housing 244 and the test tube 320 results in a sealing engagement 336 which prevents the passage of sperm between the outer surface 268 of the housing 244 and the inner surface 332 of the test tube 320.

The upper cavity 330 and the lower cavity 334 are placed in fluid communication by inserting a suitable media, such as HEPES, through the internal passage 266, until the fluid level is raised above the filter 315 and into the upper cavity 330. The motile sperm in the semen sample, located in the lower cavity 334, will begin to swim upwardly toward the filter 315. The filter 315 permits the motile sperm to swim upwardly therethrough (e.g., the pores 314 of the filter 315 provide passage for the motile sperm) and into the upper cavity 330 to be obtained (e.g., via a pipette) for use or storage.

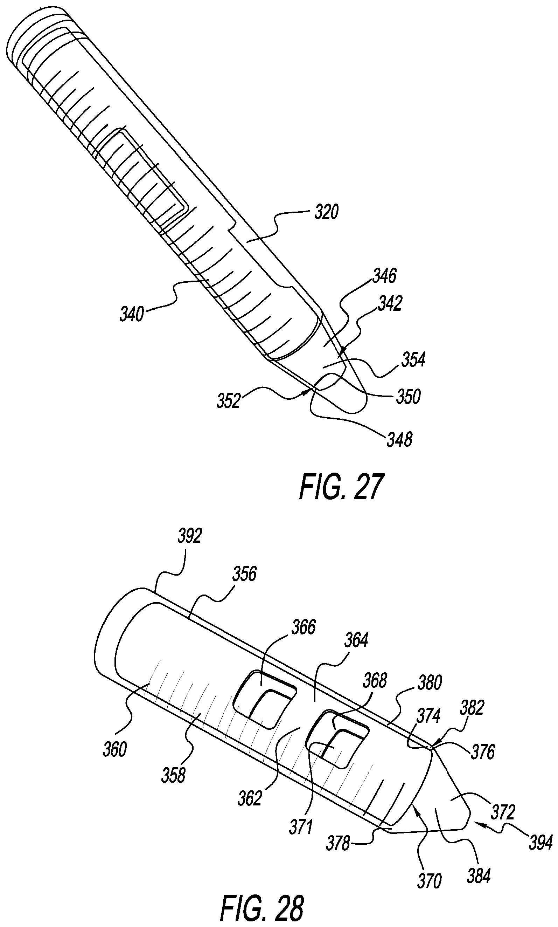

The housing 244 can be constructed to be fully located within the test tube 320 when placed in the fully inserted configuration 338. As is shown in Fig. 27, the entire housing 244 is located within the test tube 320, and the second end 248 of the housing 244 is depicted as being slightly vertically lower than the first end 322 of the test tube 320. The sperm separating device 240 is preferably a single use device (e.g., once the motile sperm have been obtained from the upper cavity 330, the test tube 320, housing 244, and remaining biologics and fluids (e.g., semen, sperm, and media) can be discarded.

Referring now to Fig. 27, a further exemplary form of a test tube insertable motility-based sperm separating device 340 will now be described. Referring now to Figs. 26-27, the sperm separating device 340 is similar to the sperm separating device 240 of Fig. 26; therefore, only those distinguishing features will be described. The sperm separating device 340 is positioned within the same test tube 320 (e.g., as has been previously described).

A lower portion 342 of the sperm separating device 340 includes a frusto-conical extension 346. This frusto-conical extension 346 extends down into the conical form 328 of the test tube 320, below the conical transition 339. When the sperm separating device 340 is fully inserted into the test tube 340, as shown in Fig. 27, an outer surface 348 of the lower portion 342 contacts and sealingly engages 352 with an inner surface 350 of the conical form 328 of the test tube 320 (e.g., so that sperm are not able to pass upwardly between the sperm separating device 340 and the test tube 320).

A filter (not shown) is retained in the frusto-conical extension 346 of the lower portion 342 at a location internal to 354. This configuration (e.g., having the filter located lower in the conical form 328) has been found to be beneficial for smaller semen samples. The sperm separating device 340 is a preferred form for bovine sperm that is typically packaged and sold in .25 mL quantities (often frozen). Positioning the filter toward the smaller semen sample eases use and extraction of motile sperm (e.g., the motile sperm are not overly diluted with media).

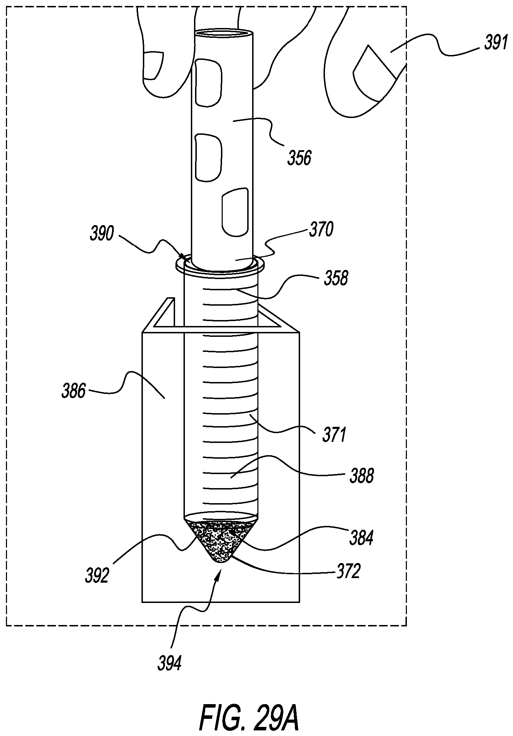

Referring now to Figs. 28-29D, a further exemplary form of a test tube insertable motility-based sperm separating device 356 will now be described. The sperm separating device 356 is similar to the sperm separating device 240 of Fig. 21; however, the sperm separating device 356 and conical 372 test tube 358 are significantly larger. This conical test tube 358 has a 50 mL capacity 360, which is believed to be well suited for porcine semen samples (e.g., due to the large volume of semen to separated). The test tube 358 extends between a first end 392 having an opening 390, and a closed second end 394 including the conical 372 form.

The sperm separating device 356 includes a housing 362, which is depicted as including a substantially hollow cylindrical form 364. Viewing windows 366, 368 can be located in the outer wall 380 of the housing 362. A passageway 381 extends internal to the outer wall 380 of the housing 362. The passageway 381 completely extends through the housing 362 (e.g., from the upper portion 396 to the lower portion 370). A sperm separating member, depicted as filter 393, is positioned in the passageway 381 toward the lower portion 370.

When the housing 362 is fully inserted into the test tube 358, the filter 393 serves as a divider or partition between the lower cavity 384 (e.g., the semen sample 388 holding area) and the upper cavity 371. As shown in Fig. 28, when the housing 362 is fully inserted into the test tube 358, the outer surface 376 of the lower portion 370 of the housing 362 is closely received by the inner surface 374 of the test tube 358 on or near the conical transition 378. The lower portion 370 of the housing 362 preferably sealingly engages 382 with the test tube 358, preventing motile sperm from bypassing the filter 393.

Referring now to Figs. 29A-29D, while keeping Fig. 28 in mind, an exemplary method of utilizing the sperm separating device 356 to obtain progressively motile sperm 395 from a semen sample 388 will now be described.

A sperm sample 388 is obtained and is placed in the test tube 358. The sperm sample 388 is depicted as a porcine sample; however, the sperm separating devices of the present application may be utilized to separate motile sperm from semen samples for a variety of species (e.g., human, bovine, porcine, equine, etc.).

The test tube 358 can be placed in a test tube holder 386 to ease insertion of the sperm separating device 356 and to reduce the likelihood of spillage. Fig. 29A depicts a user 391 grasping the upper portion 396 of the sperm separating device 356 to position the lower portion 370 into the opening 390 in the test tube 358. It will be appreciated to a person of skill in the art that the user 391 will wear gloves (not shown) to prevent contamination to the semen sample 388 and the progressively motile sperm cells 395 separated therefrom.

Advantageously, the upper portion 396 of the sperm separating device 356 can serve as an insertion tool, enabling a user to manipulate the sperm separating device 356 to insert the sperm separating device 356 into the test tube. The longitudinal sizing of the housing 362 relative the test tube 358 enables the user 391 to interact with the upper portion 396 to align and insert the lower portion 370 of the housing 362 into the opening 390 of the test tube 358.

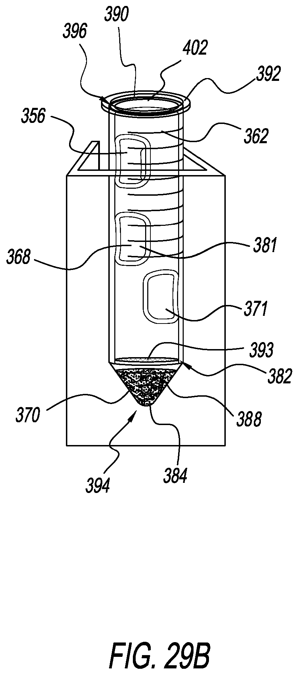

Once the lower portion 370 is aligned with the opening 390, the user 391 can insert the housing 362 into the test tube 358 (e.g., via pressing downwardly on the upper portion 396 and/or guiding the housing 362 downwardly depending on the clearance between the housing 362 and the test tube 358). The sperm separating device 356 is fully inserted into the test tube 358 when sealing engagement 382 is present, as shown in Fig. 29B. The upper portion 396 of the sperm separating device 356 is depicted as being substantially flush with the first end of the test tube 392, when fully inserted therein.

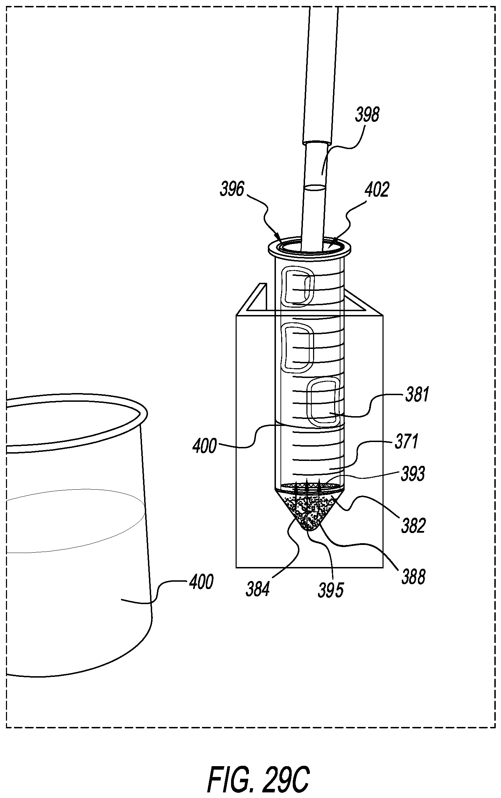

The filter 393 is positioned between the lower cavity 384, in which the porcine semen sample 388 is located, and the upper cavity 371. A suitable semen preparation media 400 (e.g., HEPES, MOPS, various bicarbonate solutions, or other desired semen preparation media) is then inserted through the opening 402, as shown in Fig. 29C. A pipette 398 can be utilized to insert the media 400, with the pipette 398 being inserted through the opening and into the passageway 381. The media 400 will then flow downardly through the passageway 381, through the filter 393, and into the lower cavity 384 including the semen sample 388.

A suitable volume of media 400 will elevate the fluid level to a location in the upper cavity 371, above the filter 393. The media 400 places the lower cavity 384 in fluid communication with the upper cavity 371, with the filter 393 serving to partition and separate the upper cavity 371 and the lower cavity 384.

Once a suitable volume of media 400 has been inserted the test tube 358, and the sperm separating device 356 located therein, can be placed in an incubator. While the user 391 performs other tasks, the progressively motile sperm cells 395 within the lower cavity 384 will begin to swim upwardly toward the filter 393.

As has been described with regard to filter 315 of Fig. 24, pores in the filter 393 provide passage for progressively motile sperm cells 395 to swim through the filter 393 and into the upper cavity 371. Advantageously, the filter 393 restricts the passage of non-progressively motile sperm and almost entirely prevents the passage of non-motile sperm therethrough.

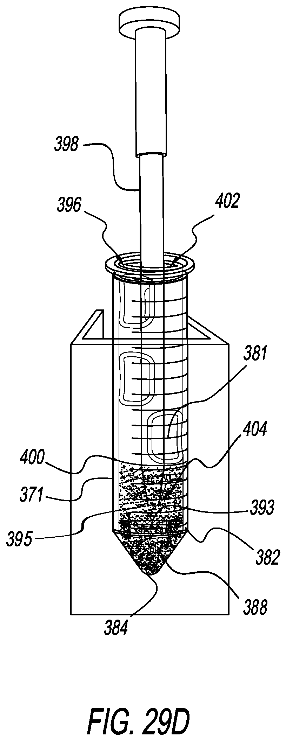

Referring now to Fig. 29D, after an appropriate incubation period (e.g., which can depend upon the specific species of sperm utilized, the media utilized, as well as the filter 393 utilized), many progressively motile sperm 395 will have swum through the filter 393 and into the upper cavity 371. A pipette 398 can be inserted through the passageway 381 and into the upper cavity 371. The pipette 391 can be utilized to draw up 404 media 400 from the upper cavity 371, with progressively motile sperm 395 therein. The progressively motile sperm 395 can be directly utilized for assistive reproductive technologies (e.g., artificial insemination, IVF, etc.) or can be further processed and packaged to freeze for future use.

Preferably, the sperm separating device 356 is a single-use device; therefore, after the desired progressively motile sperm 395 are retrieved, the test tube 358 and the sperm separating device 356 can be discarded. However, it is also contemplated that the sperm separating device 356 can be constructed from suitable materials such that it could be sterilized and reused.

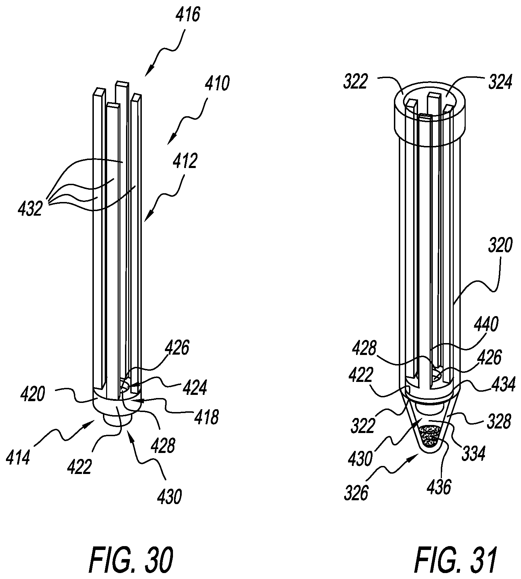

Fig. 30 depicts another exemplary form of a test tube insertable motility-based sperm separating device 410. The sperm separating device 410 includes a body 412 which extends between a lower portion 414 and an upper portion 416. The body 412 includes a housing 418 which houses a filter 426.

The housing 418 has a hollow cylindrical form 420. The filter 416 is positioned in an internal area 414 of the housing 418. The internal area 414 serves as a passageway between a lower opening 430 and an upper opening 418. The filter 416 is positioned in the internal area 414 between the lower opening 410 and the upper opening 418.

Insertion members 432 are coupled with the housing 418. A user can grasp insertion members 432 to manipulate the housing 418 and insert the housing 418 into test tube 320. An outer surface 422 of the housing 418 contacts an inner surface 434 of the test tube 320.

Fig. 31 depicts the sperm separating device 410 inserted into the test tube 320 with a semen sample 436 located in a lower cavity 442. As has been described, a media will be inserted into the test tube to provide flow communication between the lower cavity 442 and the upper cavity 440. Motile sperm within the semen sample 436 will swim upwardly toward the filter 426.

The filter 426 permits motile sperm to swim upwardly therethrough and into the upper cavity 440. The filter 426 restricts the passage of non-motile sperm to the upper cavity 440. Media, including the motile sperm therein, can be drawn from the upper cavity 440 (e.g., to be immediately utilized for assistive reproduction technologies or frozen for future use).

The filters 315, 393, and 426 can be constructed from a variety of materials and utilizing various construction techniques permit motile sperm to swim therethrough while restricting non-motile sperm. As has been briefly discussed, the species of sperm (e.g., human, bovine, equine, porcine, etc.) and sizing characteristics of that species should also be considered when determining a suitable filter construction.

The filters 315, 393, and 426 are preferably formed of a polymer material which can be woven or non-woven. In a specific, non-limiting example, the filters 315, 393, and 426 can be a Jomesa^{®} 10 µm PET woven filter membrane, a Jomesa^{®} 15 µm woven PET filter membrane, a Jomesa^{®} 20 µm PET woven filter membrane, Novorosort manufactured woven PET filter membranes, pluriSelect^{®} manufactured woven PET filter membranes, Cytiva^{®} manufactured 10 µm etched PC filter membranes, LensHooke^{®} etched filter membranes, Zymot^{®} etched PC filter membranes, as well as other woven, non-woven, foamed, etched, or layered filter membranes.

As was described with filter 315, the filters 393 and 426 can include pore sizes in the range of 5 µm to 100 µm. It is believed that pore sizes greater than 5 µm and less than 30 µm can provide adequate separation for human, bovine, and porcine sperm (e.g., motile sperm can swim upwardly through the filters 393 and 426 but non-motile sperm is restrained from passing therethrough).

The following table depicts characteristics of the motile sperm obtained during preliminary testing of a prototype of semen separating device 240, with three exemplary constructions of filter 274 (e.g., a Cytiva^{®} manufactured 10 µm etched PC filter membrane, a Jomesa^{®} 15 µm woven PET filter membrane, and a Jomesa^{®} 20 µm PET filter membrane).

| Sample | Semen Count (million/mL) | % Motility | % Normal Morphology |
|---|---|---|---|
| Base line | 52.0 | 73 % | 1 % |
| Cytiva 10 um | 21.0 | 90 % | 6 % |
| Jomesa 15 um | 18.5 | 75 % | 2 % |
| Jomesa 20 um | 22.5 | 88 % | 2 % |

As will be appreciated, the above results indicate that the test tube insertable motility-based semen separating device 240, with the filter 274 constructions depicted, is able to separate motile sperm from a semen sample utilizing swim-up without the detrimental effects of centrifugation.

### Embodiments

The disclosure will now further describe the following numbered embodiments. It will be appreciated that some or all of the embodiments summarize aspects of the disclosure as provided in the detailed description and the figures. Accordingly, the embodiments are also to be read in connection with the preceding paragraphs and the appended figures, and do not limit the disclosure. The features and preferences as described hereinabove apply also to the following embodiments.

Embodiment 1. A test tube insertable spermatozoa separation apparatus, comprising:
a housing having a lower portion and an upper portion, wherein the lower portion is configured for insertion into a test tube; and
a sperm separating member positioned within an internal area of the lower portion of the housing, wherein the sperm separating member is configured to permit motile spermatozoa to swim upwardly therethrough toward the upper portion of the housing.

Embodiment 2. The spermatozoa separation apparatus of embodiment 1, wherein the sperm separating member is further configured to restrict the passage of non-motile spermatozoa.

Embodiment 3. The spermatozoa separation apparatus of embodiment 2, wherein an outer portion of the housing is structured to closesly engage with an inner wall of the test tube.

Embodiment 4. The spermatozoa separation apparatus of embodiment 2 or 3, wherein the housing includes an outer wall defining a cylindrical shell-like form, wherein the internal area is an internal passageway that extends from the lower portion to the upper portion, and wherein the sperm separating member is located within the internal passageway.

Embodiment 5. The spermatozoa separation apparatus of embodiment 4, wherein the outer wall includes a plurality of viewing windows.

Embodiment 6. The spermatozoa separation apparatus of any of embodiments 1-5, wherein the sperm separating member includes a plurality of microfluidic channels.

Embodiment 7. The spermatozoa separation apparatus of any of embodiments 1, wherein the sperm separating member is a sperm separating filter having a pore size greater than 5 µm and less than 30 µm.

Embodiment 8. The spermatozoa separation apparatus of embodiment 7, wherein the filter is formed of at least one of nylon, polyethylene terephthalate, and polycarbonate.

Embodiment 9. A test tube insertable spermatozoa swim-up separation device, comprising:
a housing having an internal passageway extending therethrough, wherein a lower portion of the housing is configured for insertion into a test tube; and
a sperm separating filter located within the internal passageway, wherein the sperm separating filter is configured to permit motile spermatozoa to swim upwardly therethrough while restricting the passage of non-motile spermatozoa.

Embodiment 10. The device of embodiment 9, wherein the housing includes a hollow cylindrical form.

Embodiment 11. The device of embodiment 10, further comprising a retention ring positioned within the internal passageway, wherein the retention ring retains the sperm separating filter in the internal passageway, and wherein an outer circumference of the retention ring engages with the housing.

Embodiment 12. The device of embodiment 10 or 11, wherein the sperm separating filter is located toward the lower portion of the housing, wherein, upon insertion of the lower portion of the housing into the test tube the sperm separating filter divides the test tube into an upper cavity and a lower cavity, and wherein the sperm separating filter provides passage for the motile spermatozoa to swim upwardly from the lower cavity into the upper cavity.

Embodiment 13. The device of any of embodiments 9-12, wherein the housing is formed of crystal polystyrene or polycarbonate.

Embodiment 14. The device of any of embodiments 9-13, wherein the sperm separating filter includes a pore size greater than 5 µm and less than 30 µm. Embodiment 15. The device of embodiment 14, wherein the sperm separating filter is formed of polyethylene terephthalate or etched polycarbonate.

Embodiment 16. A motility-based spermatozoa separation apparatus, comprising:
a hollow cylindrical body having an internal passageway, wherein the hollow cylindrical body is configured for insertion into a test tube;
a sperm separating member positioned in the internal passageway; and
wherein, upon insertion of the hollow cylindrical body into the test tube, the sperm separating member separates the test tube into a lower cavity and an upper cavity in fluid communication with the lower cavity, and wherein the sperm separating member provides passage for motile spermatozoa to swim upwardly from the lower cavity and into the upper cavity.

Embodiment 17. The spermatozoa separation apparatus of embodiment 16, wherein the sperm separating member restricts the passage of non-motile spermatozoa therethough.

Embodiment 18. The spermatozoa separation apparatus of embodiment 16 or 17, wherein the sperm separating member is a filter.

Embodiment 19. The spermatozoa separation apparatus of embodiment 16, 17 or 18, wherein the filter is one of a polyethylene terephthalate filter and an etched polycarbonate filter, and wherein the filter includes a pore size greater than 5 µm and less than 30 µm.

Embodiment 20. The spermatozoa separation apparatus of embodiment 18 or 19, wherein the hollow cylindrical body extends between an upper portion and a lower portion, wherein the filter is retained in the internal passageway with a retention ring.

While the invention has been described in connection with what is presently considered to be the most practical and preferred embodiment, it is to be understood that the invention is not to be limited to the disclosed embodiment(s), but on the contrary, is intended to cover various modifications and equivalent arrangements included within the spirit and scope of the appended claims, which scope is to be accorded the broadest interpretation so as to encompass all such modifications and equivalent structures as permitted under the law.

It should be understood that while the use of the word preferable, preferably, or preferred in the description above indicates that feature so described may be more desirable, it nonetheless may not be necessary and any embodiment lacking the same may be contemplated as within the scope of the invention, that scope being defined by the claims that follow. In reading the claims it is intended that when words such as "a," "an," "at least one" and "at least a portion" are used, there is no intention to limit the claim to only one item unless specifically stated to the contrary in the claim. Further, when the language "at least a portion" and/or "a portion" is used the item may include a portion and/or the entire item unless specifically stated to the contrary.

## Claims

1. A test tube insertable spermatozoa separation apparatus, comprising:
a housing having a lower portion and an upper portion, wherein the lower portion is configured for insertion into a test tube; and
a sperm separating member positioned within an internal area of the lower portion of the housing, wherein the sperm separating member is configured to permit motile spermatozoa to swim upwardly therethrough toward the upper portion of the housing.

2. The spermatozoa separation apparatus of claim 1, wherein the sperm separating member is further configured to restrict the passage of non-motile spermatozoa.

3. The spermatozoa separation apparatus of claim 2, wherein an outer portion of the housing is structured to closesly engage with an inner wall of the test tube.

4. The spermatozoa separation apparatus of any of claims 1-3, wherein the housing has an internal passageway extending therethrough, and wherein the sperm separating filter is located within the internal passageway.

5. The spermatozoa separation apparatus of any of claims 1-4, wherein the housing includes an outer wall defining a cylindrical shell-like form.

6. The spermatozoa separation apparatus of claim 5, wherein the outer wall includes a plurality of viewing windows.

7. The spermatozoa separation apparatus of any of claims 1-6, wherein the sperm separating member includes a plurality of microfluidic channels.

8. The spermatozoa separation apparatus of any of claims 1-7, wherein the sperm separating member is a sperm separating filter.

9. The spermatozoa separation apparatus of claim 8, wherein the filter has a pore size greater than 5 µm and less than 30 µm.

10. The spermatozoa separation apparatus of claim 8 or 9, wherein the filter is formed of at least one of nylon, polyethylene terephthalate, and polycarbonate.

11. The spermatozoa separation apparatus of claim 4, or any of claims 5-10 as far as dependent from claim 4, further comprising a retention ring positioned within the internal passageway, wherein the retention ring retains the sperm separating filter in the internal passageway, and wherein an outer circumference of the retention ring engages with the housing.

12. The apparatus of any of claims 1-11, wherein the sperm separating filter is located toward the lower portion of the housing.

13. The apparatus of any of claims 1-12, wherein, upon insertion of the lower portion of the housing into a test tube the sperm separating filter divides the test tube into an upper cavity and a lower cavity, and wherein the sperm separating filter provides passage for the motile spermatozoa to swim upwardly from the lower cavity into the upper cavity.

14. The apparatus of any of claims 1-13, wherein the housing is formed of crystal polystyrene or polycarbonate.
